# EUROPEAN PATENT APPLICATION

(11) **EP 1 983 085 A1**
(43) Date of publication of application: **22.10.2008**
(21) Application number: 06835007.3
(22) Date of filing: 20.12.2006
(51) Int. Cl.: D06F 25/00, D06F 39/00, D06F 39/08, D06F 58/02

(54) **FIBER STRUCTURE TREATMENT APPARATUS**

(30) Priority: 31.01.2006 JP 2006022644; 26.07.2006 JP 2006203003; 16.11.2006 JP 2006310668
(71) Applicant: Sharp Kabushiki Kaisha, Osaka-shi, Osaka 545-8522 (JP)
(72) Inventor: IKEMIZU, Mugihei, Osaka-shi, Osaka 547-0002 (JP)
(74) Representative: Brown, Kenneth Richard
(86) International application number: PCT/JP2006/325337
(87) International publication number: WO 2007/088684

(57) **Abstract**

Provided is a fabric structure treatment apparatus in which it is not required to heat at a high temperature a fabric structure and/or components of the treatment apparatus, which are/is targeted for sterilization; in which a dry state is not necessarily required; which does not bring about deterioration, discoloring, and the like of the fabric structure and/or components of the treatment apparatus, targeted for the sterilization; the fabric structure and/or components of the treatment apparatus, targeted for the sterilization, are not damaged; and which is capable of attaining sufficient bactericidal action. A washing and drying machine (100) as the fabric structure treatment apparatus comprises: a rotating drum (3) as a container unit for containing, inside thereof, clothing (6) as the fabric structure; a silver ion water supplying unit (40) as a silver ion applying unit for applying silver ions to the rotating drum (3) and/or the clothing (6) contained inside of the rotating drum (3); and a light irradiating unit (20) as a light irradiator for irradiating with light the rotating drum (3) and/or clothing (6), to which the silver ions have been applied by the silver ion water supplying unit (40).

## Description

### TECHNICAL FIELD

The present invention relates to fabric structure treatment apparatuses and, in particular, to a fabric structure treatment apparatus, such as a washing machine, a washing and drying machine, and a drying machine, which is used for a fabric structure such as clothing, a futon, and a rug and is capable of enhancing sterilization action.

### BACKGROUND ART

Conventionally, there has been proposed a washing and drying machine, in which clothing is irradiated with light such as infrared rays having heating action in order to dry the clothing, for example, in Japanese Patent Application Laid-Open Publication No. 2005-152193 (Patent Document 1), Japanese Patent Application Laid-Open Publication No. 2005-168687 (Patent Document 2), and Japanese Patent Application Laid-Open Publication No. 2005-177081 (Patent Document 3).

In addition, there has been proposed an electric washing machine, in which ultraviolet irradiation is performed in order to decompose and remove dirt composed of organic substances adhering to a top plate of an outer tub, a water tub, an outer lid, and the like, for example, in Japanese Patent Application Laid-Open Publication No. 9-135992 (Patent Document 4). Also, there has been proposed a washing machine, in which laundry is irradiated with ultraviolet rays in order to prevent an unpleasant smell, which is caused by propagation of various bacteria, from being imparted to the laundry for example, in Japanese Patent Application Laid-Open Publication No. 10-43481 (Patent Document 5).

Furthermore, there has been proposed a washing machine, in which silver ions are applied to laundry in order to impart bactericidal action so as to kill various bacteria adhering to the laundry or in order to impart antibacterial action so as to suppress propagation of various bacteria adhering to the laundry, for example, in Japanese Utility Model Application Laid-Open Publication No. 5-74487 (Patent Document 6), Japanese Patent Application Laid-Open Publication No. 2001-276484 (Patent Document 7), Japanese Patent Application Laid-Open Publication No. 2003-290594 (Patent Document 8), Japanese Patent Application Laid-Open Publication No. 2004-215817 (Patent Document 9), and Japanese Patent Application Laid-Open Publication No. 2004-321313 (Patent Document 10).

Moreover, there has been proposed a drum-type washing and drying machine or a clothing drying apparatus, in which clothing is dried by using a heat pump unit in order to enhance a drying efficiency, for example, in Japanese Patent Application Laid-Open Publication No. 2004-229954 (Patent Document 11) and Japanese Patent Application Laid-Open Publication No. 2005-304987 (Patent Document 12).

Meanwhile, there has been proposed a washing machine, which is equipped with an illuminating lamp for illuminating an inside of a tub, for example, in Japanese Patent Application Laid-Open Publication No. 5-245292 (Patent Document 13), Japanese Patent Application Laid-Open Publication No. 6-190186 (Patent Document 14), Japanese Patent Application Laid-Open Publication No. 6-327887 (Patent Document 15), and Japanese Patent Application Laid-Open Publication No. 2000-157781 (Patent Document 16).
Patent Document 1: Japanese Patent Application Laid-Open Publication No. 2005-152193
Patent Document 2: Japanese Patent Application Laid-Open Publication No. 2005-168687
Patent Document 3: Japanese Patent Application Laid-Open Publication No. 2005-177081
Patent Document 4: Japanese Patent Application Laid-Open Publication No. 9-135992
Patent Document 5: Japanese Patent Application Laid-Open Publication No. 10-43481
Patent Document 6: Japanese Utility Model Application Laid-Open Publication No. 5-74487
Patent Document 7: Japanese Patent Application Laid-Open Publication No. 2001-276484
Patent Document 8: Japanese Patent Application Laid-Open Publication No. 2003-290594
Patent Document 9: Japanese Patent Application Laid-Open Publication No. 2004-215817
Patent Document 10: Japanese Patent Application Laid-Open Publication No. 2004-321313
Patent Document 11: Japanese Patent Application Laid-Open Publication No. 2004-229954
Patent Document 12: Japanese Patent Application Laid-Open Publication No. 2005-304987
Patent Document 13: Japanese Patent Application Laid-Open Publication No. 5-245292
Patent Document 14: Japanese Patent Application Laid-Open Publication No. 6-190186
Patent Document 15: Japanese Patent Application Laid-Open Publication No. 6-327887
Patent Document 16: Japanese Patent Application Laid-Open Publication No. 2000-157781

### DISCLOSURE OF THE INVENTION

### Problems to be solved by the invention

However, in a case where clothing is irradiated with light including infrared rays or the like having heating action in order to dry the clothing, sterilization can be performed by increasing a temperature of the clothing, whereas there arises a problem that the clothing is damaged because the clothing is heated at a high temperature. Also in this case, because the sterilization treatment for the clothing is not performed when the clothing is not dried, there arises a problem that the sterilization treatment cannot be performed for clothing or the like for which washing is performed but drying is unwanted by using the washing and drying machine.

In addition, in a case of a washing machine in which ultraviolet irradiation is performed for the sterilization treatment, there arises problems that a synthetic resin and the like of which the washing machine is composed are deteriorated due to the ultraviolet irradiation and that discoloring or yellowing of laundry such as clothing is caused due to the ultraviolet irradiation.

Furthermore, in a case of a washing machine in which silver ions are applied to laundry, because it is required to apply silver ions of greater than or equal to a prescribed amount in order to attain desired antibacterial action and bactericidal action, there arises a problem that sufficient antibacterial action and bactericidal action cannot be attained depending on an amount of the laundry, a volume of used water, etc.

Moreover, in a case of a drum-type washing and drying machine or a clothing drying apparatus in which clothing is dried by using a heat pump unit in order to enhance a drying efficiency, because clothing is dried at a low temperature, there may be a case where a temperature within the machine or the apparatus does not reach a temperature of approximately 65°C to 80°C at which various bacteria are killed. For this reason, in the case where the clothing is dried by using the heat pump unit, there may be a case where a sterilization effect attained by heating at the low temperature becomes insufficient.

Therefore, an object of the present invention is to provide a fabric structure treatment apparatus in which it is not required to heat at a high temperature a fabric structure and/or components of the treatment apparatus, which is/are targeted for sterilization; in which a dry state is not necessarily required; in which no deterioration and no discoloring of the fabric structure and/or components of the treatment apparatus, targeted for the sterilization, are brought about; in which the fabric structure and/or components of the treatment apparatus, targeted for the sterilization, are not damaged; and which is capable of attaining sufficient bactericidal action.

### Means for solving the problems

A fabric structure treatment apparatus according to the present invention comprises: a container unit for containing, inside thereof, a fabric structure to which silver ions have been applied; and a light irradiating unit for irradiating with light the fabric structure to which the silver ions have been applied.

In the fabric structure treatment apparatus according to the present invention, the fabric structure to which the silver ions have been previously applied is further irradiated with the light, whereby bactericidal action can be enhanced. In addition, the fabric structure targeted for sterilization is not heated, a dry state is not necessarily required, and the fabric structure to which the silver ions have been applied is merely irradiated with the light, whereby the bactericidal action can be enhanced. Further, the fabric structure to which the silver ions have been applied is merely irradiated with the light, whereby propagation of microorganisms such as various bacteria can be more effectively suppressed. As compared with an apparatus in which only silver ions are applied to a fabric structure, the fabric structure treatment apparatus according to the present invention allows desired bactericidal action to be attained by using a small amount of silver. Accordingly, a fabric structure such as clothing, a futon, and a rug to which the silver ions have been previously applied by a washing machine, a washing and drying machine, or the like which has a function of applying the silver ions can be sterilized, a material targeted for sterilization is not damaged, and sufficient bactericidal action can be attained.

A fabric structure treatment apparatus according to the present invention comprises: a container unit for containing, inside thereof, a fabric structure; a silver ion applying unit for applying silver ions to the container unit and/or the fabric structure contained in the container unit; and a light irradiating unit for irradiating with light the container unit and/or fabric structure to which the silver ions have been applied by the silver ion applying unit.

In the fabric structure treatment apparatus according to the present invention, the container unit and/or fabric structure to which the silver ions have been applied is irradiated with the light, whereby the bactericidal action can be enhanced. In addition, the container unit and/or fabric structure targeted for sterilization is/are not heated, a dry state is not necessarily required, and the container unit and/or fabric structure to which the silver ions have been applied is/are merely irradiated with the light, whereby the bactericidal action can be enhanced. Further, the container unit and/or fabric structure to which the silver ions have been applied is merely irradiated with the light, whereby propagation of microorganisms such as various bacteria can be more effectively suppressed. As compared with an apparatus in which only silver ions are applied to a fabric structure, the fabric structure treatment apparatus according to the present invention allows a desired bactericidal action to be attained by using a small amount of silver. Accordingly, components, made of a synthetic resin or the like, of which the container unit is composed and/or a fabric structure such as clothing, a futon, and a rug can be sterilized, a material targeted for the sterilization is not damaged, and sufficient bactericidal action can be attained.

In the fabric structure treatment apparatus according to the present invention, it is preferable that the light irradiating unit emits light including visible light. In this case, the irradiation of the light including the visible light is performed, whereby the bactericidal action can be further enhanced, and damages of the material such as deterioration and discoloring due to ultraviolet rays can be prevented.

In the fabric structure treatment apparatus according to the present invention, it is preferable that a washing process of washing the fabric structure is performed and after the washing process, the light irradiating unit irradiates with the light the container unit and/or fabric structure to which the silver ions have been applied by the silver ion applying unit. In this case, the light irradiating unit irradiates with the light the container unit and/or fabric structure at least after the washing process, whereby the fabric structure targeted for washing and/or container unit containing the fabric structure targeted for washing can be sterilized, a material targeted for sterilization is not damaged, and sufficient bactericidal action can be attained.

In the fabric structure treatment apparatus according to the present invention, it is preferable that a rinsing process of rinsing the fabric structure is performed; during the rinsing process, the silver ion applying unit applies the silver ions to the container unit and/or fabric structure; and after the rinsing process, the light irradiating unit irradiates with the light the container unit and/or fabric structure to which the silver ions have been applied by the silver ion applying unit. In this case, the silver ion applying unit is capable of applying the silver ions to the container unit and/or fabric structure during the rinsing process and at least after the rinsing process, the light irradiating unit irradiates with the light the container unit and/or fabric structure, whereby the fabric structure targeted for washing and/or the container unit containing the fabric structure targeted for washing can be sterilized, a material targeted for the sterilization is not damaged, and the sufficient bactericidal action can be attained.

In the fabric structure treatment apparatus according to the present invention, it is preferable that a dewatering process of removing water from the fabric structure is performed; and during the dewatering process and/or after the dewatering process, the light irradiating unit irradiates with the light the container unit and/or fabric structure, to which the silver ions have been applied by the silver ion applying unit. In this case, the dewatering process of removing the water from the fabric structure is performed and during the dewatering process and/or after the dewatering process, the light irradiating unit irradiates with the light the container unit and/or fabric structure, whereby the fabric structure targeted for washing and/or the container unit containing the fabric structure targeted for washing can be sterilized, a material targeted for the sterilization is not damaged, and sufficient bactericidal action can be attained. In addition, because during the dewatering process and/or after the dewatering process, the light irradiating unit irradiates with the light the container unit and/or fabric structure, washing water or rinsing water is not present in the container unit. Therefore, the fabric structure targeted for washing and/or the container unit containing the fabric structure targeted for washing can be efficiently irradiated with the light.

In the fabric structure treatment apparatus according to the present invention, it is preferable that a drying process of drying the fabric structure is performed; and during the drying process and/or after the drying process, the light irradiating unit irradiates with the light the container unit and/or fabric structure, to which the silver ions have been applied by the silver ion applying unit. In this case, the drying process of drying the fabric structure is performed; and during the drying process and/or after the drying process, the light irradiating unit irradiates with the light the container unit and/or fabric structure, to which the silver ions have been applied by the silver ion applying unit, whereby the fabric structure targeted for washing and/or the container unit containing the fabric structure targeted for washing can be sterilized, a material targeted for the sterilization is not damaged, and sufficient bactericidal action can be attained. In addition, because the drying process and/or after the drying process, the light irradiating unit irradiates with the light the container unit and/or fabric structure, no water is present in the container unit. Therefore, the fabric structure as a material targeted for drying and/or the container unit containing the material targeted for drying can be efficiently irradiated with the light.

In the fabric structure treatment apparatus according to the present invention, it is preferable that the drying process is performed at a low temperature. In this case, the fabric structure is dried at the low temperature, whereby damaging the fabric structure, caused by the drying, can be prevented and bactericidal action can be enhanced under a condition of the low temperature.

Further in the fabric structure treatment apparatus according to the present invention, it is preferable that the above-mentioned drying process is performed by using a heat pump unit. In this case, in the fabric structure treatment apparatus such as the drying machine and the washing and drying machine in which drying at a low temperature is performed by using the heat pump unit, bactericidal action can be enhanced under a condition of the low temperature.

In the fabric structure treatment apparatus according to the present invention, it is preferable that after the fabric structure has been taken out from the container unit, the light irradiating unit irradiates with the light the container unit. In this case, after the fabric structure has been taken out from the container unit, an inside of the container unit can be effectively sterilized.

In the fabric structure treatment apparatus according to the present invention, it is preferable that the silver ions are silver ions generated through electrolysis. In this case, bactericidal action due to the light irradiation can be enhanced.

A fabric structure treatment apparatus according to the present invention which uses water containing silver ions and comprises a light irradiating unit for irradiating the water with light.

Through doing this in the above-mentioned manner, the water used for washing and rinsing is caused to contain the silver ions and the water is irradiated with the light, whereby rinsing water and drying water can be effectively sterilized.

### EFFECT OF THE INVENTION

According to the present invention, it is not required to heat at a high temperature a container unit and/or a fabric structure; a dry state is not necessarily required; deterioration, discoloring, and the like of the container unit and/or fabric structure targeted for sterilization are not brought about; the container unit and/or fabric structure, targeted for sterilization, is/are not damaged; in a fabric structure treatment apparatus, for example, a washing machine, a drying machine, or the like which treats a fabric structure such as clothing, a futon, and a rug, a sterilization effect can be enhanced in a simple process; and sufficient bactericidal action can be attained.

### BRIEF DESCRIPTION OF THE DRAWINGS

Fig. 1 is a graph showing a relationship between silver amounts applied to test samples and the numbers of bacteria existing in the test samples.
Fig. 2 is a graph showing a relationship between illuminances of a white fluorescent lamp and the numbers of bacteria existing in the test samples.
Fig. 3. is one sectional side view showing a schematic configuration of a washing and drying machine as one embodiment of a fabric structure treatment apparatus according to the present invention.
Fig. 4 is another sectional side view showing a schematic configuration of a washing and drying machine as one embodiment of the fabric structure treatment apparatus according to the present invention.
Fig. 5 is a flow chart showing a whole laundering process.
Fig. 6 is a flow chart showing a washing process.
Fig. 7 is a flow chart showing a rinsing process.
Fig. 8 is a flow chart showing a silver sterilization rinsing process.
Fig. 9 is a flow chart showing a dewatering and drying selection process.
Fig. 10 is a flow chart showing a dewatering process.
Fig. 11 is a flow chart showing a light irradiation dewatering process.
Fig. 12 is a flow chart showing a drying process.
Fig. 13 is a flow chart showing a light irradiation drying process.
Fig. 14 is a flow chart showing a tub inside sterilization process.
Fig. 15 is a graph showing a relationship between time lapses and the numbers of bacteria existing in the test samples.
Fig. 16 is a graph showing a relationship between kinds of a method of generating silver ions to be applied and the numbers of bacteria existing in the test samples.
Fig. 17 is a graph showing a relationship between silver ion concentrations with and without light irradiation and the numbers of bacteria.

### EXPLANATION OF REFERENCE NUMERALS

1: main body, 2: water tub, 3: rotating drum, 6: clothing, 20: light irradiating unit, 21: light source, 40: silver ion water supplying unit, 100: washing and drying machine.

### BEST MODE FOR CARRYING OUT THE INVENTION

Based on the examination of the above-mentioned problematic points of the background art, the present inventors have devoted themselves to studies, investigating a sterilization method using a fabric structure treatment apparatus from various aspects. As a result, the present inventors have found that when a container unit and/or a fabric structure, to which silver ions have been applied, are/is further irradiated with light, bactericidal action can be enhanced. Based on such findings obtained by the present inventors, the present invention was made.

A washing machine, a washing and drying machine, a drying machine or the like as one embodiment of a fabric structure treatment apparatus according to the present invention comprises: a silver ion water supplying unit or the like as a silver ion applying unit for applying silver ions to a container unit, which contains a fabric structure such as clothing, a futon, and a rug and is referred to as an outer tub, a water tub, a washing tub, a dewatering bin, a washing and dewatering bin, a rotating tub, a rotating drum, or the like, and/or to the fabric structure contained in the container unit; and a light irradiating unit for irradiating with light the container unit and/or fabric structure to which the silver ions have been applied by the silver ion applying unit.

In the one embodiment of the fabric structure treatment apparatus according to the present invention, enhancement of bactericidal action is enabled by further irradiating with the light the container unit and/or fabric structure to which the silver ions have been applied. In addition, without heating the container unit and/or fabric structure targeted for sterilization in a drying process or the like and without necessarily having to render the container unit and/or fabric structure targeted for the sterilization in a dry state, the enhancement of the bactericidal action is enabled only by irradiating with the light the container unit and/or fabric structure to which the silver ions have been applied. Furthermore, effective suppression of propagation of various bacteria or the like is enabled only by irradiating with the light the container unit and/or fabric structure to which the silver ions have been applied. Meanwhile, the fabric structure treatment apparatus according to the present invention makes it possible to attain desired bactericidal action by using a small amount of silver, as compared with the conventional washing machine, the conventional washing and drying machine, or the like in which application of the silver ions to the container unit and/or fabric structure is merely performed. Accordingly, components of which the container unit made of a synthetic resin and the like is composed and/or the fabric structure such as clothing, a futon, a rug and the like can be sterilized; a material targeted for the sterilization is not damaged; and sufficient bactericidal action can be obtained.

The silver ion water supplying unit or the like as the silver ion applying unit is, for example, a unit with which the silver ions are added to washing water and the silver ions are applied to laundry as the fabric structure and parts of a tub as the container unit which contact the washing water. Specifically, the silver ions are eluted from an electrolysis-type silver ion elution unit to water supplied when final rinsing is performed, thereby applying the silver ions to the container unit and/or fabric structure.

The washing water is, for example, a fluid at large, such as water used for washing and rinsing and cooling water used for dehumidifying, which is used in the washing machine as one example of the fabric structure treatment apparatus. With the washing water containing the silver ions, processes of washing and rinsing are performed, thereby enabling the application of the silver ions to the laundry and an inside of the tub of the washing machine.

The application of the silver ions to the container unit and/or fabric structure may be performed, for example, by immersing a material in not only the water containing the silver ions but also a liquid such as a solvent as well as a supercritical fluid or the like or by spraying a small amount of a liquid such as the water containing the silver ions in an atomized manner. The liquid such as the water containing the silver ions may be prepared through electrolysis using electrodes containing silver. As a liquid other than the liquid prepared through the electrolysis, the liquid such as the water containing the silver ions may be prepared by using a silver-ion-containing substance having a structure in which silver ions can be released or dissolved by immersing the substance in a liquid such as water. As a specific example of the silver-ion-containing substance, zeolite, silicagel, glass, calcium phosphate, zirconium phosphate, silicate, titanium oxide, whisker, ceramics, etc., which supports the silver ions, or a resin, a fabric, or the like which contains these substances can be cited. In addition, the liquid such as the water containing the silver ions may be prepared, for example, by dissolving a silver compound such as a silver nitrate and a silver chloride in a liquid.

As the light irradiating unit, a fluorescent lamp, an electric bulb, a light emitting diode (LED), etc. can be used. The light irradiating unit may be structured so as to include a light source, such as a bactericidal lamp and an ultraviolet (UV) lamp, which mainly includes light in an ultraviolet region; a light source, such as a black light capable of applying near ultraviolet rays, which mainly includes light in a near ultraviolet region; or a light source, such as a halogen heater, which mainly includes light in an infrared region. However, it is preferable that the light irradiating unit is structured by using a light source, such as a fluorescent lamp, an incandescent lamp, a light emitting diode (LEDs having colors such as white, blue, red, and green), and a visible light laser, which mainly includes visible light.

In the one embodiment of the fabric structure treatment apparatus according to the present invention, the light irradiating unit emits light mainly including the visible light, thereby allowing further enhancement of the bactericidal action and enabling the prevention of deterioration, yellowing, discoloring, and the like of the material due to ultraviolet rays.

In addition, it is preferable that in the washing machine, the washing and drying machine, or the like as the one embodiment of the fabric structure treatment apparatus according to the present invention, a washing process of washing the fabric structure such as clothing, a futon, and a rug is performed and after the washing process, the light irradiating unit irradiates with the light the container unit, which is referred to as the outer tub, the water tub, the washing tub, the dewatering bin, the washing and dewatering bin, the rotating tub, the rotating drum, or the like, and/or the fabric structure, to which the silver ions have been applied by the silver ion applying unit. In this case, the light irradiating unit irradiates with the light the container unit and/or the fabric structure at least after the washing process, whereby the sterilization of the fabric structure targeted for washing and/or the container unit containing the fabric structure targeted for washing is enabled; the material targeted for the sterilization is not damaged; and sufficient bactericidal action can be obtained.

Further, it is preferable that in the washing machine, the washing and drying machine, or the like as the one embodiment of the fabric structure treatment apparatus according to the present invention, a rinsing process of rinsing the fabric structure such as clothing, a towel, a futon, and a rug is performed; during the rinsing process, the silver ion applying unit applies the silver ions to the container unit, which is referred to as the outer tub, the water tub, the washing tub, the dewatering bin, the washing and dewatering bin, the rotating tub, the rotating drum, or the like and/or to the fabric structure; and after the rinsing process, the light irradiating unit irradiates with the light the container unit and/or fabric structure to which the silver ions have been applied by the silver ion applying unit. In this case, the silver ion applying unit is capable of applying the silver ions to the container unit and/or fabric structure during the rinsing process and the light irradiating unit irradiates with the light the container unit and/or fabric structure at least after the rinsing process, whereby the sterilization of the fabric structure targeted for washing and/or the container unit containing the fabric structure targeted for washing is enabled; the material targeted for the sterilization is not damaged; and sufficient bactericidal action can be obtained.

Furthermore, it is preferable that in the washing machine, the washing and drying machine, or the like as the one embodiment of the fabric structure treatment apparatus according to the present invention, a dewatering process of removing water from the fabric structure such as clothing, a futon, and a rug is performed; and during and/or after the dewatering process, the light irradiating unit irradiates with the light the container unit, which is referred to as the outer tub, the water tub, the washing tub, the dewatering bin, the washing and dewatering bin, the rotating tub, the rotating drum, or the like, and/or the fabric structure, to which the silver ions have been applied by the silver ion applying unit. In this case, the dewatering process of removing water from the fabric structure is performed; and during and/or after the dewatering process, the light irradiating unit irradiates with the light the container unit and/or fabric structure, whereby the sterilization of the fabric structure targeted for washing and/or the container unit containing the fabric structure targeted for washing is enabled; the material targeted for the sterilization is not damaged; and sufficient bactericidal action can be obtained. In addition, because during and/or after the dewatering process, the light irradiating unit irradiates with the light the container unit and/or fabric structure, washing water or rinsing water is not present in the container unit, whereby efficient light irradiation of the fabric structure targeted for washing and/or the container unit containing the fabric structure targeted for washing is enabled. In particular, because during the washing process and the rinsing process, water is retained in the tub, the material targeted for washing cannot be irradiated with the light. However, after having drained water in the dewatering process, the light irradiation is performed, thereby enabling effective light irradiation of the material targeted for washing.

It is preferable that in the drying machine, the washing and drying machine, or the like as the one embodiment of the fabric structure treatment apparatus according to the present invention, a drying process of drying the fabric structure such as clothing, a futon, and a rug is performed; and during and/or after the drying process, the light irradiating unit irradiates with the light the container unit, which is referred to as the outer tub, the water tub, the washing tub, the dewatering bin, the washing and dewatering bin, the rotating tub, the rotating drum, a drying chamber or the like, and/or the fabric structure, to which the silver ions have been applied by the silver ion applying unit. In this case, the drying process of drying the fabric structure is performed and during and/or after the drying process, the light irradiating unit irradiates with the light the container unit and/or fabric structure, whereby the sterilization of the fabric structure targeted for washing and/or the container unit containing the fabric structure targeted for washing is enabled; the material targeted for the sterilization is not damaged; and sufficient bactericidal action can be attained. In addition, because during and/or after the drying process, the light irradiating unit irradiates with the light the container unit and/or fabric structure, washing water or rinsing water is not present in the container unit of the washing and drying machine or water is not present in the container unit of a drying-dedicated machine, whereby efficient light irradiation of the fabric structure targeted for drying and/or the container unit containing the fabric structure targeted for drying is enabled.

In a case of the fabric structure treatment apparatus which is capable of performing the laundering process and the drying process, when the drying process is performed, the light irradiating unit may perform the light irradiation during and/or after the drying process; and when the drying process is not performed, the light irradiating unit may perform the light irradiation during and/or after the dewatering process. This allows duplication of the light irradiation to be avoided.

In general, in a case of a drying machine, a washing and drying machine, or the like which has a water-cooling dehumidification mechanism, moisture in a dehumidifying unit where a temperature is low is removed through condensation of moisture in the air and thereafter, the obtained air is heated in a high-temperature unit, thereby lowering a relative humidity. The obtained air whose temperature is high and whose humidity is low is caused to contact laundry, whereby the moisture is caused to move from the laundry to a side of the air. From the moist air obtained in such a manner, the moisture is removed in the dehumidifying unit. By repeating this, drying is implemented. Accordingly, in the drying machine having the water-cooling dehumidification mechanism, the greater the difference between an amount of a saturated water vapor in the dehumidifying unit where a temperature is low and an amount of a saturated water vapor in the high-temperature unit where a temperature is high, the higher the efficiency. Therefore, the greater the difference between an air temperature in the dehumidifying unit and an air temperature in the high-temperature unit, the better.

For example, in a case of water-cooling dehumidification in which tap water is used for the dehumidification, a temperature at which the dehumidification is performed is dependent on a water temperature. For this reason, in the summer when the water temperature sometimes exceeds 30°C, there may be a case where a dehumidification effect is not sufficient.

Consequently, it is considered that a heat pump unit is adopted in order to perform a drying process at a low temperature in the drying machine, the washing and drying machine, or the like as the one embodiment of the fabric structure treatment apparatus according to the present invention. In a case of the drying machine, the washing and drying machine, or the like in which the heat pump unit is adopted, because it is possible to set a temperature in the dehumidifying unit so as to be lower than the water temperature, an air temperature in the dehumidifying unit can be made low, as compared with the water-cooling dehumidification. Therefore, even when an air temperature in the high-temperature unit is made low, highly efficient drying can be performed. Note that even in the drying machine, the washing and drying machine, or the like having the water-cooling dehumidification mechanism, drying at a low temperature is made possible by performing the drying process for a long time. The low temperature in this case is approximately 30°C to 80°C and more preferably, is approximately 40°C to 65°C at which the fabric structure is less damaged and a period of time required for drying is not excessively long.

As another embodiment of the fabric structure treatment apparatus according to the present invention, a drying machine, a washing and drying machine, or the like which performs the drying process at the above-mentioned low temperature may comprise a silver ion applying unit and a light irradiating unit. Many microorganisms are killed at a temperature 65°C to 80°C. However, when the drying process is performed at the low temperature, there may be a case where a temperature in the drying machine or the washing and drying machine does not reach the above-mentioned temperature, whereby a sterilization effect is likely to be insufficient. In the fabric structure treatment apparatus according to the present invention, even when the drying process is performed at the low temperature, enhancement of the bactericidal action is enabled only by irradiating with the light the container unit and/or fabric structure to which the silver ions have been applied; and more effective suppression of propagation of the microorganisms such as various bacteria is enabled only by irradiating with the light the container unit and/or fabric structure to which the silver ions have been applied.

In addition, when a light irradiating unit such as an LED and a fluorescent lamp is used at a high temperature, a lifetime thereof is shortened. In particular, in a case of the LED, when a temperature is high, a light output is reduced and a luminous efficiency is reduced, thereby resulting in a problem that a power consumption is increased. However, through the light irradiation at the low temperature, curbing the above-mentioned problem is enabled; prolonging a lifetime of the light irradiating unit is enabled; and lowering a power consumption is enabled.

Preferably, the light irradiation is performed at a temperature, for example, of 30°C to 80°C and further preferably, the light irradiation is performed at a temperature of 40°C to 65°C. In addition, the fabric structure is dried at the low temperature in the drying process performed in the drying machine, the washing and drying machine, or the like as the one embodiment of the fabric structure treatment apparatus according to the present invention, whereby damaging the fabric structure due to drying can be prevented and under a condition of the low temperature, enhancement of the bactericidal action is enabled.

Note that in the one embodiment of the fabric structure treatment apparatus according to the present invention, the light irradiating unit may irradiate the container unit with the light after the fabric structure has been taken out from the container unit.

Specifically, for example, in the washing machine, the washing and drying machine, or the like as the one embodiment of the fabric structure treatment apparatus, the light irradiation is stopped after a lapse of a predetermined time period following the end of a laundering operation or a drying operation which is performed after a rinsing process in which silver ions are applied to the container unit. Thus, in a case where it is desired to sterilize an inside of the tub, the light irradiation for the inside of the tub can be efficiently performed for a predetermined time period after laundry has been taken out. In addition, because the light irradiation is stopped after the lapse of the predetermined time period, it is not necessary for a user to stand by until the light irradiation is finished. At this time, for example, by rotating the container unit such as a rotating drum, a space inside of the container unit may be irradiated with the light in an even manner. In addition, by using a material, of a whole or a part of the container unit, which has a function of reflecting light emitted from light irradiating means, the space inside of the container unit can be irradiated with the light in a further even manner. In particular, for example, in a case where a moving unit such as the rotating drum is formed by using the material having the function of reflecting light and is further provided with projections and depressions, light can be reflected in various directions, thereby attaining more effective light irradiation.

In addition, a door of the container unit such as the rotating drum is opened, the laundry is taken out, the door is closed, and thereafter, the light irradiation may be started. This allows the light irradiation to be started in a space closed after the laundry has been taken out.

Further, not only after finishing the laundering operation or the drying operation but also at least after finishing the rinsing process in which the silver ions are applied to the container unit, the door of the washing machine, the washing and drying machine, or the like is opened in order to take out the laundry from the container unit, and thereafter, the light irradiation may be started. This allows the light irradiation to be implemented even in a case where a user does not close the door after having taken out the laundry. At this time, in order to avoid any problem arising when a user is exposed to the light, it is preferable that the emitted light is visible light.

In the fabric structure treatment apparatus according to the present invention, it is preferable that the container unit and/or fabric structure are/is irradiated with the light of greater than or equal to a predetermined illuminance. In this case, the material is irradiated with the light of greater than or equal to the predetermined illuminance, whereby further enhancement of the bactericidal action is enabled.

Hereinafter, as one embodiment of a fabric structure treatment apparatus according to the present invention, a washing and drying machine will be described with reference to figures.

Hereinafter, the one embodiment according to the present invention will be described with reference to the figures.

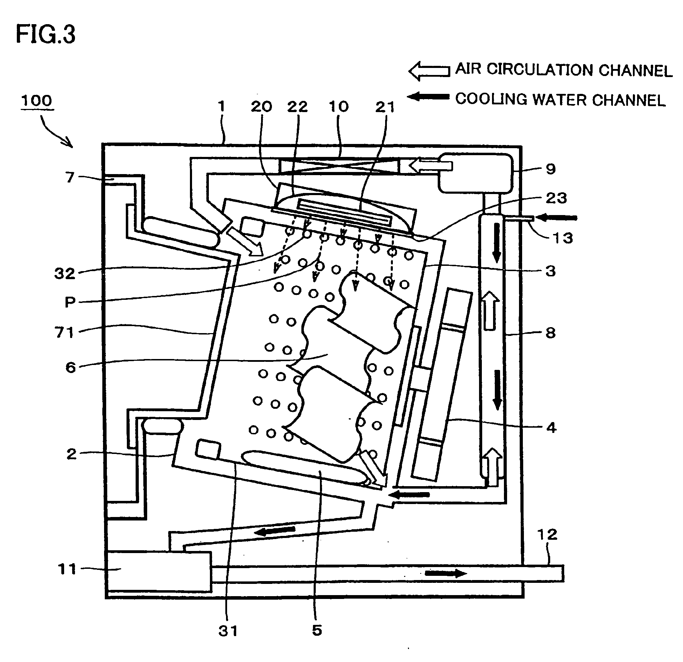

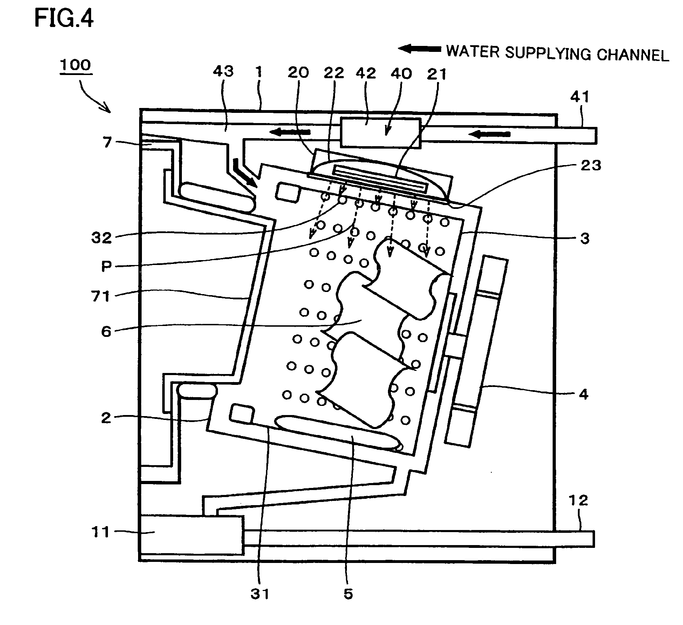

Fig. 3 is one sectional side view showing a schematic configuration of the washing and drying machine as the one embodiment of the fabric structure treatment apparatus. Fig. 4 is another sectional side view thereof.

As shown in Fig. 3 and Fig. 4, the washing and drying machine 100 comprises: a main body 1; a water tub 2, as a container unit, which is fixed inside of the main body 1; and a rotating drum 3, as a container unit, which is supported in a rotatable manner inside of the water tub 2.

At the bottom of the rotating drum 3, a rotation mechanism unit which rotates the rotating drum 3 in a normal and reverse manner by using a motor 4 is attached. A baffle 5 for stirring clothing in the rotating drum 3 is attached on an inner circumference wall surface of the rotating drum 3. A door unit 7 which allows taking out and putting in clothing 6 as a fabric structure and keeps air tightness and water tightness is attached to an opening portion of the rotating drum 3 in an openable and closable manner. A door glass 71 which allows a state of the clothing 6 to be observed externally is provided on the door unit 7.

As shown in Fig. 3, a drying apparatus comprises a circulation fan 9 and an air heater 10. The air heater 10 heats the air. The circulation fan 9 circulates the air in communication with an inside of the water tub 2, an inside of the rotating drum 3, and a cooler 8 as a channel through which dehumidification is performed such that the air heated by the air heater 10 evaporates moisture of the clothing.

A light irradiating unit 20 as a light irradiator includes a light source 21, which is a fluorescent lamp, an incandescent lamp, an LED, or the like, for emitting light mainly including visible light; is installed in the water tub 2; and is capable of directly irradiating with the light an inner circumference wall surface of the water tub 2, an outer circumference wall surface and an inner circumference wall surface 31 of the rotating drum 3, and the clothing 6 in a direction indicated by an arrow P. A plurality of small holes 32 piercing the rotating drum 3 to an inside thereof are formed on the outer circumference wall surface and the inner circumference wall surface 31 of the rotating drum 3 such that the light emitted from the light source 21 can pass therethrough and the clothing 6 can be irradiated with the light. The light irradiating unit 20 has a reflecting plate 22 provided therein in order to reflect the light emitted from the light source 21 in a direction toward the outer circumference wall surface and the inner circumference wall surface 31 of the rotating drum 3. In addition, the light irradiating unit 20 has provided therein a heat-resistant glass plate 23 having a high light transmission property in order to protect the light source 21 from washing water and water resulting from dewatering. The light irradiating unit 20 is installed on the outer circumference wall surface of the water tub 2 such that the reflecting plate 22 is a back surface thereof and that the heat-resistant glass plate 23 is firmly fixed on the outer circumference wall surface of the water tub 2. Specifically, the light irradiating unit 20 is fixed on the outer circumference wall surface of the water tub 2 with screws or the like in a state where the water tub 2 and the heat-resistant glass plate 23 are sealed by using a sealant, an adhesive, or the like having a heat-resisting property (thermal insulation property) and a water-proofing property.

In the present embodiment, the light irradiating unit is installed on the water tub 2. However, it is only required to install the light irradiating unit at a position where the container unit and/or fabric structure can be irradiated with the light. For example, the light irradiating unit may be installed at a portion of the door of the container unit so as to irradiate the container unit with the light.

As shown in Fig. 4, a silver ion applying unit 40 comprises: a water supplying pipe 41; a silver ion elution unit 42 connected to the water supplying pipe 41; and a water supplying channel 43 connecting between the silver ion elution unit 42 and a water supplying inlet of the water tub 2. Inside of the silver ion elution unit 42, two platy silver electrodes are disposed in a spaced manner. Water is supplied into an inside of the silver ion elution unit 42 through the water supplying pipe 41 and a voltage is applied between the silver electrodes, whereby the silver ions are eluted into the inside of the silver ion elution unit 42. The water containing the silver ions is supplied into the inside of the water tub 2 through the water supplying channel 43. This causes the silver ion water to be applied to the inner circumference wall surface of the water tub 2 as the container unit and the outer circumference wall surface and the inner circumference wall surface 31 of the rotating drum 3 as the container unit as well as the clothing 6 as the fabric structure.

Next, an outline of an operation of the washing and drying machine according to the present invention structured as described above will be described.

A signal, not shown, from a control unit rotates the motor 4 in a normal and reverse manner and the rotating drum 3 directly connected to the motor 4 rotates similarly in the normal and reverse manner. This rotation causes the baffle 5, fixedly mounted on the inner circumference wall surface of the rotating drum 3, to repeat operations of lifting up and dropping down the clothing 6, thereby performing a washing process. In such a manner, the washing process is performed by utilizing the so-called beating-washing effect.

Upon finishing the washing process, a drain pump 11 is driven, thereby draining water in the water tub 2 from a drain hose 12. Thereafter, the operation proceeds to a rinsing process and a dewatering process, thereby finishing the laundering process.

When a drying operation is started after having finished the laundering process, the normal and reverse rotation of the rotating drum 3 causes stirring action generated by an up-and-down movement of the clothing 6, and concurrently, power is supplied to the circulation fan 9 and the air heater 10, whereby an air temperature rises. This heated air evaporates moisture from the clothing and the evaporated moisture flows into the cooler 8. Because in the cooler 8, cooling water has been supplied from a cooling water inlet pipe 13 which is disposed at an upper position of the cooler 8 in a facing manner, the evaporated moisture is cooled and condensed. The condensed moisture flows into a side of the drain pump 11, is mixed with the cooling water, and is drained. The dehumidified air is refluxed again into the air heater 10 and heated. The heated air flows into the rotating drum 3, heats the clothing 6, and evaporates moisture contained in the clothing 6. By repeating the above-described operation, drying of the clothing 6 proceeds. A source for heating the clothing, used in this drying process, is the air heater 10. In general, the air heater 10 is mounted inside of an air circulation duct and a sheathed heater which is formed by coating a resistance heating wire with metal is used as the air heater 10.

Next, an operation of the washing and drying machine 100 will be described step by step with reference to flow charts.

First, as shown in Fig. 3 and Fig. 4, a user opens the door unit 7 and puts the clothing 6 as the laundry in the rotating drum 3 and a detergent in a detergent compartment (not shown) in the water supplying inlet. If necessary, the user puts a finishing agent in a finishing agent compartment (not shown). The finishing agent may be put therein during the laundering process.

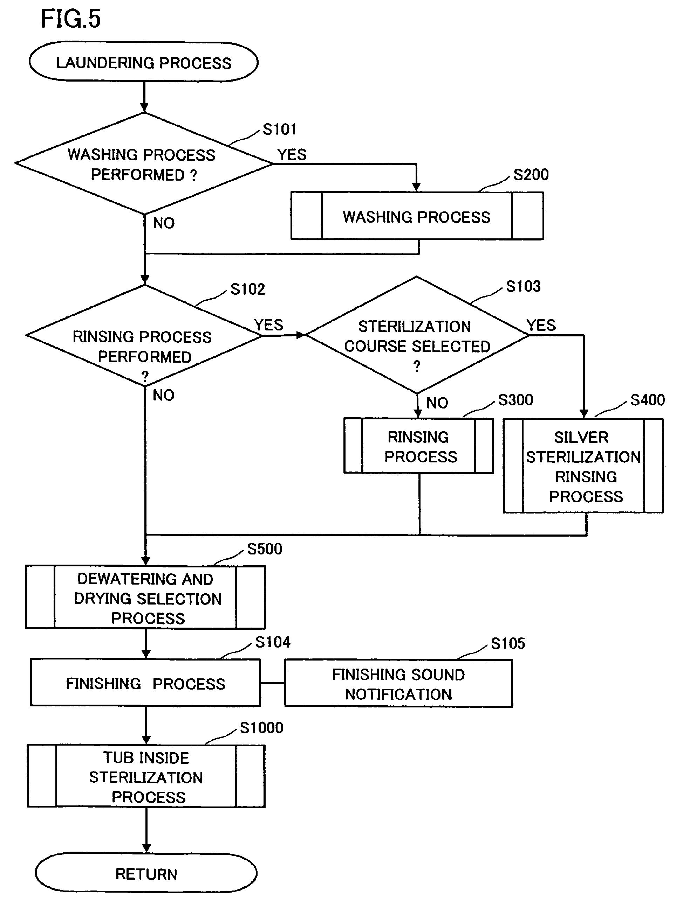

After having made the preparation of loading of the detergent, the user closes the door unit 7 and operates operation buttons (not shown) of an operation switch unit on an operation panel to select a laundering condition (laundering mode). Finally, when the user presses a start button, the control unit (not shown) carries out a laundering process corresponding to the above-mentioned laundering mode in accordance with the flow charts shown in Fig. 5 through Fig. 14.

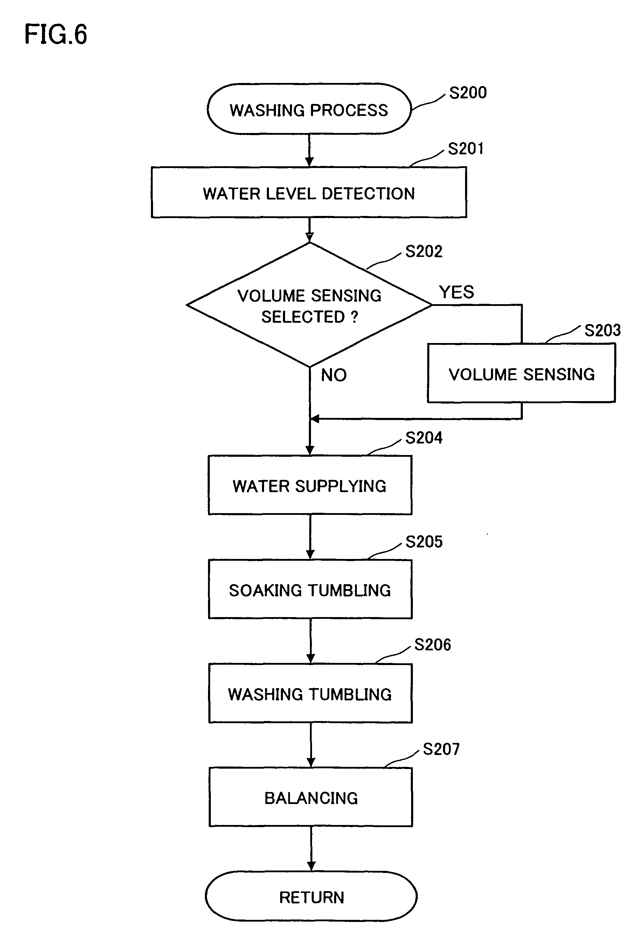

Fig. 5 is the flow chart showing a whole laundering process. As shown in Fig. 5, first at step S101, it is determined whether a washing process has been selected. When selected, the laundering process proceeds to step S200. What is performed in the washing process at step S200 will be separately described with reference to the flow chart shown in Fig. 6. After having finished the washing process, the laundering process proceeds to step S102. On the other hand, when it is determined at step S101 that the washing process has not been selected, the laundering process proceeds directly to step S102.

At step S102, it is determined whether a rinsing process has been selected. When selected, the laundering process proceeds to step S103.

At step S103, it is determined whether a sterilization course has been selected. When selected, the laundering process proceeds to step S400. On the other hand, when it is determined at step S103 that the sterilization course has not been selected, the laundering process proceeds to step S300.

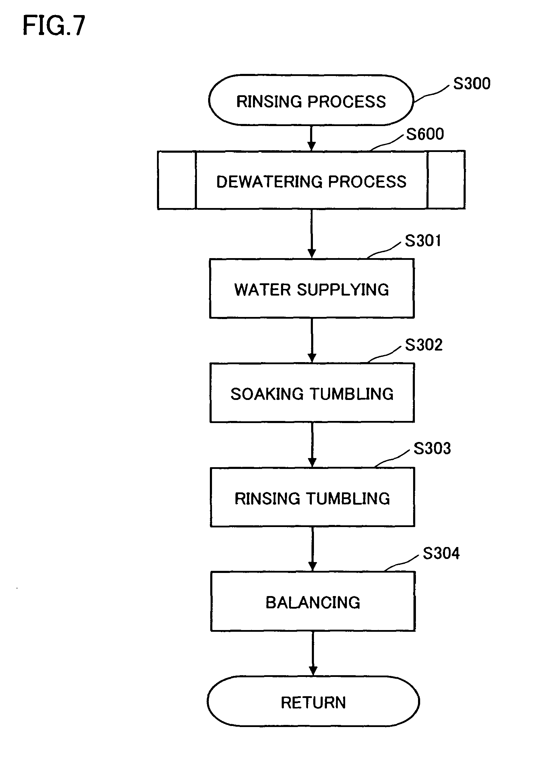

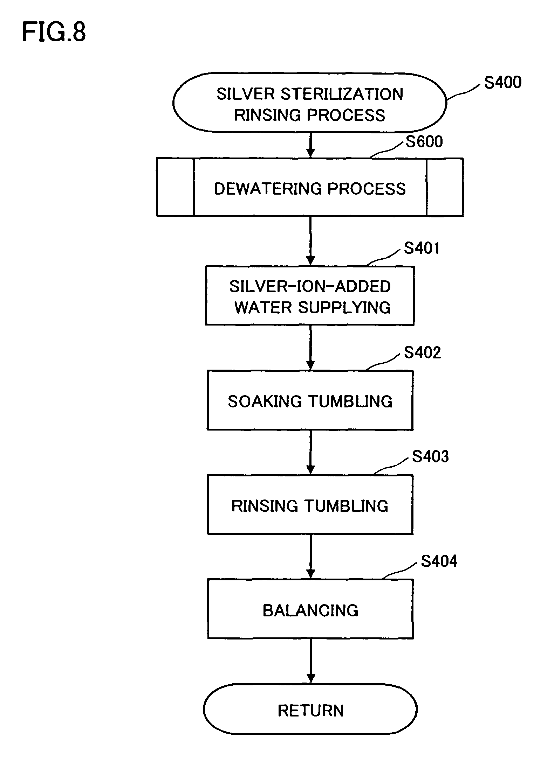

What is performed in the silver sterilization rinsing process at step S400 will be separately described with reference to the flow chart shown in Fig. 8. What is performed in the rinsing process at step S300 will be separately described with reference to the flow chart shown in Fig. 7. Note that the rinsing process may be performed a plurality of times. After having finished the rinsing process, the laundering process proceeds to step S500. On the other hand, when it is determined at step S102 that the rinsing process has not been selected, the laundering process proceeds directly to step S500.

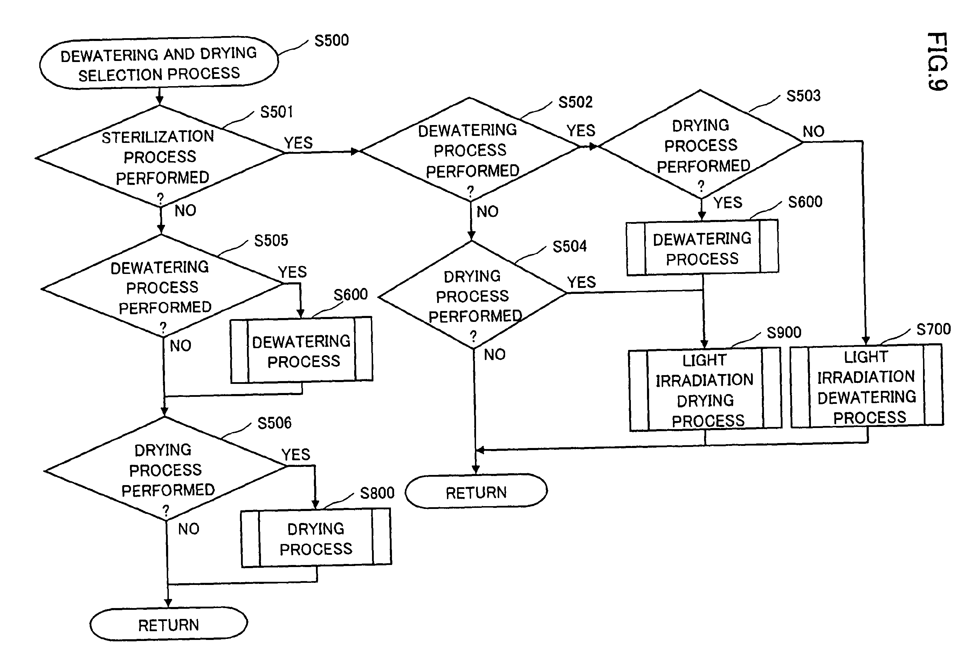

What is performed in the dewatering and drying selection process at step S500 will be separately described with reference to the flow chart shown in Fig. 9. After having finished the dewatering and drying selection process, the laundering process proceeds to step S104.

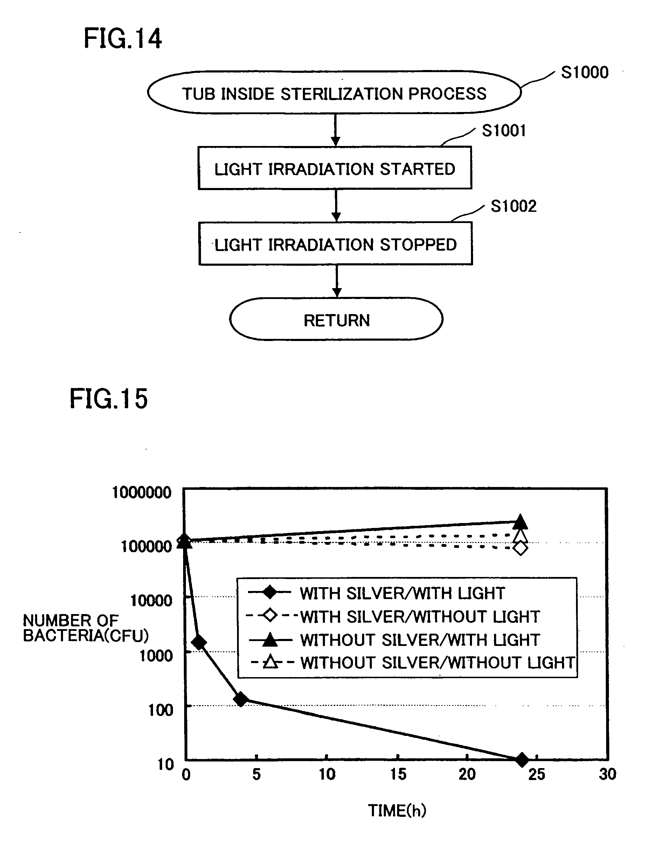

At step S104, a finishing process performed by the control unit automatically proceeds in accordance with procedures. In addition, at step S105, the control unit informs the user, with finishing sound, that the laundering process has been completed. After having finished all of the laundering process, the user opens the door unit 7, takes out the laundry, and closes the door unit 7. Thereafter, the washing and drying machine 100 returns to a waiting state in preparation for a next laundering process. However, when the sterilization course has been selected, a tub inside sterilization process at step S1000 is performed and thereafter, the washing and drying machine 100 returns to a waiting state in preparation for a next laundering process. What is performed in the tub inside sterilization process at step S1000 will be separately described with reference to the flow chart shown in Fig. 14.

Next, the respective washing process, rinsing process, silver sterilization rinsing process, dewatering and drying selection process, tub inside sterilization process in the above-described laundering process will be described in detail with reference to Fig. 6 through Fig. 14.

First, the washing process will be described.

Fig. 6 is the flow chart showing the washing process. As shown in Fig. 6, at step S201, data of a water level in the rotating drum 3, which is detected by a water level sensor, is captured. At step S202, it is determined whether volume sensing has been selected. When selected, the washing process proceeds to step S203. At step S203, the volume sensing by which a volume of the laundry is measured by utilizing a rotational load exerted on the rotating drum 3 is performed. After having performed the volume sensing, the washing process proceeds to step S204. On the other hand, when it is determined at step S202 that the volume sensing has not been selected, the washing process proceeds directly to step S204.

At step S204, a main water-feed valve (not shown) is opened and water is poured in the rotating drum 3 through a main water supplying pipe and the water supplying inlet. Accurately, the water is poured in the water tub 2 and the water enters the rotating drum 3 through the small holes 32. The detergent which has been put in the detergent compartment in the water supplying inlet is mixed with the water and is put into the rotating drum 3. At this time, the drain valve has been closed. When the water level sensor detects a set water level, the main water-feed valve is closed. The washing process proceeds to step S205.

At step S205, soaking tumbling is performed. In this soaking tumbling, the rotating drum 3 rotates at a low speed and the water is sufficiently soaked into the clothing 6 by taking out the clothing 6 from the water and dropping again the clothing 6 into the water. The air trapped in various portions of the clothing 6 is also released.

After the soaking tumbling, the washing process proceeds to step S206. At step S206, the rotating drum 3 rotates in a pattern of washing tumbling in which the rotating drum 3 lifts up high and drops the clothing 6. An impact occurring when the clothing 6 is dropped generates a jet of water in fabric of the clothing 6, whereby the clothing 6 is washed.

After a period of the washing tumbling has passed, the washing process proceeds to step S207. At step S207, the rotating drum 3 slowly rotates. In a case where the rotating drum 3 slowly rotates, the clothing 6 leaves the rotating drum 3 when the clothing 6 is at a low position and is dropped before the clothing 6 is lifted up so as to come at a high position.

Here, if the clothing 6 is dropped from the high position, the clothing 6 is slammed down on the inner circumference wall surface of the rotating drum 3 and is kept flat against the inner circumference wall surface. Therefore, when the rotating drum 3 starts dewatering rotation at a high speed, it is difficult to eliminate unbalance.

In contrast to this, when the clothing 6 leaves the inner circumference wall surface of the rotating drum 3 when the clothing is at the low position, it looks like the clothing 6 is rolled over, rather than being slammed down, and pieces of the clothing 6 are piled up in a comparatively fluffy manner. In this state, when the rotating drum 3 starts the dewatering rotation at the high speed, it is easy for the pieces of the clothing 6 to be scattered in every direction. In other words, it is easy to achieve a balance. For this reason, the pieces of the clothing 6 are disentangled by slowly rotating the rotating drum 3 in order to prepare for the dewatering rotation.

Next, with reference to the flow chart shown in Fig. 7, the rinsing process will be described.

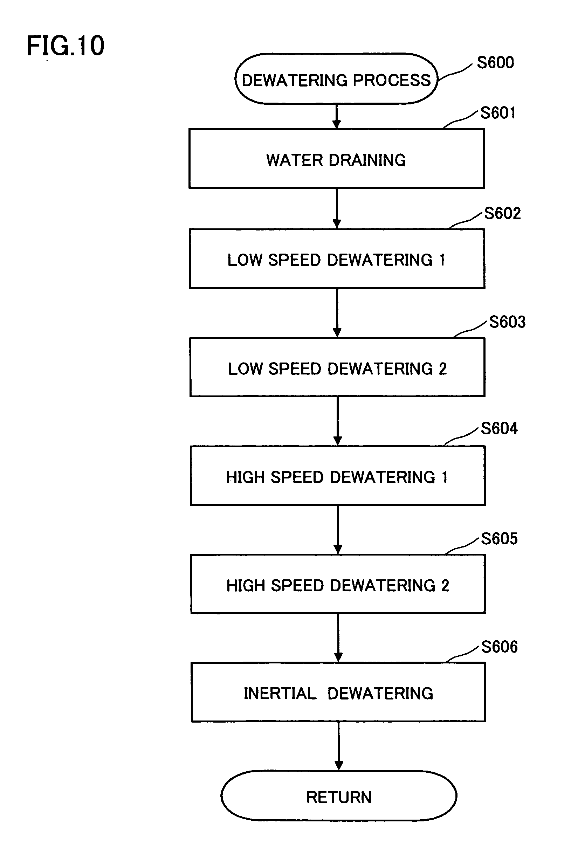

First, the dewatering process at step S600 (here, which is referred to as an intermediate dewatering process since this dewatering process is performed during the rinsing process) is performed. This dewatering process at step S600 will be described with reference to the flow chart shown in Fig. 10. After having finished the intermediate dewatering process at step S600, the rinsing process proceeds to step S301. At step S301, the main water-feed valve is opened and water is supplied up to a set water level.

After having supplied the water, the rinsing process proceeds to step S302. At step S302, soaking tumbling is performed. The soaking tumbling is the same as that performed at step S205 during the washing process.

After the soaking tumbling, the rinsing process proceeds to step S303. In accordance with a user's setting, the rotating drum 3 rotates in a pattern of rinsing tumbling. The rotating drum 3 immerses the clothing 6 in the water through the rotation thereof and lifts up high and drops down the clothing 6, thereby rinsing the clothing 6.

After a period of the rinsing tumbling has passed, the rinsing process proceeds to step S304. At step S304, the rotating drum 3 slowly rotates and disentangles the pieces of the clothing 6 in order to prepare for the dewatering rotation.

In the above description, "stored-water rinsing", which is rinsing with water stored in the rotating drum 3, is performed. However, "pouring-water rinsing", which is rising with fresh water constantly being fed, or "shower rinsing", which is rinsing with water showered on the clothing 6, may be performed.

Next, the silver sterilization rinsing process will be described with reference to the flow chart shown in Fig. 8. A point at which the silver sterilization rinsing process is different from the rinsing process at step S300 shown in Fig. 7 is that after having finished the intermediate dewatering process at step S600, the silver sterilization rinsing process proceeds to step S401; at step S401, the water-feed valve is opened; and water, which is silver ion water, is supplied up to a set water level through the water supplying pipe 41. As shown in Fig. 4, the water is supplied through the water supplying pipe 41 into an inside of the silver ion elution unit 42 and a voltage is applied between the silver electrodes, whereby the silver ions are eluted into the inside of the silver ion elution unit 42. The water containing the silver ions is supplied into the inside of the water tub 2 through the water supplying channel 43. This causes the silver ion water to be applied to the inner circumference wall surface of the water tub 2 as the container unit, the outer circumference wall surface and inner circumference wall surface 31 of the rotating drum 3 as the container unit and to the clothing 6 as the fabric structure.

The other steps S402 through S404 in the silver sterilization rinsing process are the same as those at S302 through S304 in the rinsing process.

Next, with reference to the flow chart shown in Fig. 9, the dewatering and drying selection process will be described. As shown in Fig. 9, first at step S501, it is determined whether the sterilization treatment has been selected. When selected, the dewatering and drying selection process proceeds to step S502. On the other hand, at step S501, when the sterilization treatment has not been selected, the dewatering and drying selection process proceeds to step S505.

At step S502, it is determined whether the dewatering process has been selected. When selected, the dewatering and drying selection process proceeds to step S503.

At step S503, it is determined whether the drying process has been selected. When selected, the dewatering and drying selection process proceeds to step S600.

What is performed in the dewatering process at step S600 will be separately described with reference to the flow chart shown in Fig. 10. After having finished the dewatering process, the dewatering and drying selection process proceeds to step S900. On the other hand, at step S503, when the drying process has not been selected, the dewatering and drying selection process proceeds to step S700.

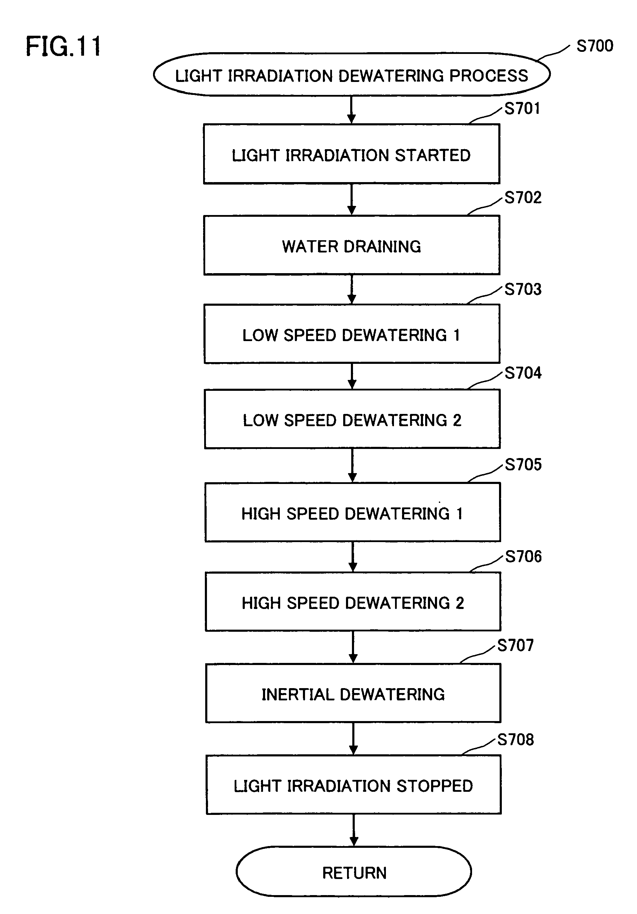

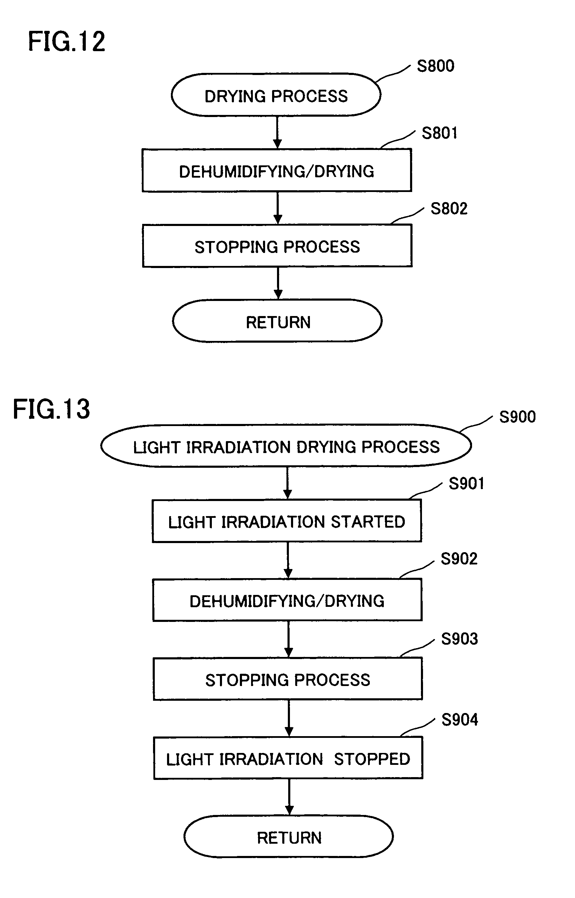

What is performed in a light irradiation dewatering process at step S700 will be separately described with reference to the flow chart shown in Fig. 11. What is performed in a light irradiation drying process at step S900 will be separately described with reference to the flow chart shown in Fig. 13.

At step S502, when the dewatering process has not been selected, the dewatering and drying selection process proceeds to step S504.

At step S504, it is determined whether the drying process has been selected. When selected, the dewatering and drying selection process proceeds to step S900. On the other hand, at step S504, when the drying process has not been selected, the dewatering and drying selection process is finished.

At step S505, it is determined whether the dewatering process has been selected. When selected, the dewatering and drying selection process proceeds to step S600.

What is performed in the dewatering process at step S600 will be separately described with reference to the flow chart shown in Fig. 10. After having finished the dewatering process, the dewatering and drying selection process proceeds to step S506. On the other hand, at step S505, when the dewatering process has not been selected, the dewatering and drying selection process proceeds directly to step S506.

At step S506, it is determined whether the drying process has been selected. When selected, the dewatering and drying selection process proceeds to step S800.

What is performed in the drying process at step S800 will be separately described with reference to the flow chart show in Fig. 12. After having finished the drying process, the dewatering and drying selection process is finished. On the other hand, at step S506, when the drying process has not been selected, the dewatering and drying selection process is finished.

Next, what is performed in the dewatering process will be described with reference to the flow chart shown in Fig. 10.

First, at step S601, the drain valve is opened. This causes the washing water or the rinsing water in the rotating drum 3 to be drained through the drain valve. The drain valve remains open during the dewatering process.

When a predetermined time period has passed and most of the water has been drained from the clothing 6, the rotating drum 3 starts the dewatering rotation. When the rotating drum 3 rotates at a high speed, the clothing 6 is pressed against the inner circumference wall surface of the rotating drum 3 due to a centrifugal force. This causes the water contained in the clothing 6 to be collected on the inner circumference wall surface of the rotating drum 3 and to be discharged from the small holes 32. The washing water or rinsing water having left the small holes 32 is pelted on the inner circumference wall surface of the water tub 2, runs along the inner circumference wall surface of the water tub 2, and streams down onto a bottom portion of the water tub 2. The water passes through the drain hose 12 and is drained outside of the main body 1.

In a sequence shown in Fig. 10, a dewatering operation at a comparatively low speed is performed at step S602 and step S603 and a dewatering operation at a comparatively high speed is performed at step S604 and step S605. After step S605, the dewatering process proceeds to step S606. At step S606, energization of the motor 4 is shut off without braking, and the rotating drum 3 is rotated by inertia and naturally stopped.

Next, what is performed in the light irradiation dewatering process will be described with reference to the flow chart shown in Fig. 11. A point at which the light irradiation dewatering process is different from the dewatering process at step S600 shown in Fig. 10 is that before draining the water at step S702, the light irradiation is started at step S701 and after inertial dewatering at step S707, the light irradiation is stopped at step S708. At step S701, as shown in Fig. 3, the control unit starts the light irradiation such that from the light source 21 composed of the fluorescent lamp, the incandescent lamp, an LED, or the like which emits light mainly including visible light, the light irradiating unit 20 as the light irradiator directly irradiates with the light the inner circumference wall surface of the water tub 2, the outer circumference wall surface and inner circumference wall surface 31 of the rotating drum 3, and the clothing 6 in the direction indicated by the arrow P. The other steps S702 through S707 in the light irradiation dewatering process are the same as those at steps S601 through S606 in the dewatering process. Upon finishing the dewatering process, at step S708, the control unit finishes the light irradiation. As described above, the inner circumference wall surface of the water tub 2, the outer circumference wall surface and inner circumference wall surface 31 of the rotating drum 3, and the clothing 6, to which the silver ion water has been applied, are irradiated with the light during the dewatering process, thereby enabling the sterilization treatment for the inner circumference wall surface of the water tub 2, the outer circumference wall surface and inner circumference wall surface 31 of the rotating drum 3, and the clothing 6. Note that after the dewatering process at steps S702 through S707, the light irradiation may be performed at step S701 and step S708. In this case, it is preferable that the rotating drum 3 is rotated in an intermittent manner.

Next, with reference to the flow chart shown in Fig. 12, what is performed in the drying process will be described. As shown in Fig. 12, dehumidifying and drying are performed at step S801. Specifically, as shown in Fig. 3, the normal and reverse rotation of the rotating drum 3 causes stirring action generated by an up-and-down movement of the clothing 6, and concurrently, power is supplied to the circulation fan 9 and the air heater 10, whereby an air temperature rises. This heated air evaporates moisture from the clothing and the evaporated moisture flows into the cooler 8. Because in the cooler 8, the cooling water has been supplied from a cooling water inlet pipe 13, the evaporated moisture is cooled and condensed. The condensed moisture flows into a side of the drain pump 11, is mixed with the cooling water, and is drained. The dehumidified air is refluxed again into the air heater 10 and heated. The heated air flows into the rotating drum 3, heats the clothing 6, and evaporates moisture contained in the clothing 6. By repeating the above-described operation, drying of the clothing 6 proceeds.

The washing and drying machine 100 has the water-cooling dehumidification mechanism and the dehumidifying and drying are performed in the above-described manner. However, instead of the water-cooling dehumidification mechanism, the washing and drying machine 100 may have a heat pump unit in order to perform the drying process at a low temperature. In the case of the washing and drying machine having the heat pump unit adopted therein, because it is possible to set a temperature in the dehumidifying unit so as to be lower than the water temperature, an air temperature in the dehumidifying unit can be made low, as compared with the water-cooling dehumidification. Therefore, even when an air temperature in the high-temperature unit is made low, highly efficient drying can be performed. Note that even in the washing and drying machine 100 having the water-cooling dehumidification mechanism, drying at a low temperature is made possible by performing the drying process for a long time. The low temperature in this case is approximately 30°C to 80°C and preferably, is approximately 40°C to 65°C.

After performing the dehumidifying and drying for a predetermined time period, a stopping process is performed at step S802 and the drying process is finished.

Next, with reference to the flow chart shown in Fig. 13, what is performed in the light irradiation drying process will be described. A point at which the light irradiation drying process is different from the drying process at step S800 shown in Fig. 12 is that before the dehumidifying and drying at step S902, the light irradiation is started at step S901 and after the stopping process at step S903, the light irradiation is stopped at step S904. At step S901, as shown in Fig. 3, the control unit starts the light irradiation such that from the light source 21 composed of the fluorescent lamp, the incandescent lamp, an LED, or the like which emits light mainly including visible light, the light irradiating unit 20 as the light irradiator directly irradiates with the light the inner circumference wall surface of the water tub 2, the outer circumference wall surface and inner circumference wall surface 31 of the rotating drum 3, and the clothing 6 in the direction indicated by the arrow P. The other steps S902 through S903 in the light irradiation drying process are the same as those at steps S801 through S802 in the drying process. Upon finishing the drying process, at step S904, the control unit finishes the light irradiation. As described above, the inner circumference wall surface of the water tub 2, the outer circumference wall surface and inner circumference wall surface 31 of the rotating drum 3, and the clothing 6, to which the silver ion water has been applied, are irradiated with the light during the drying process, thereby enabling the sterilization treatment for the inner circumference wall surface of the water tub 2, the outer circumference wall surface and inner circumference wall surface 31 of the rotating drum 3, and the clothing 6. Note that after the drying process at steps S902 through S903, the light irradiation may be performed at step S901 and step S904. In this case, it is preferable that the rotating drum 3 is rotated in an intermittent manner.

Lastly, with reference to the flow chart shown in Fig. 14, what is performed in the tub inside sterilization process will be described. At step S1001, the light irradiation is started and at step S1002, the light irradiation is stopped. At step S1001, as shown in Fig. 3, the control unit starts the light irradiation such that from the light source 21 composed of the fluorescent lamp, the incandescent lamp, an LED, or the like which emits light mainly including visible light, the light irradiating unit 20 as the light irradiator directly irradiates with the light the inner circumference wall surface of the water tub 2, the outer circumference wall surface and inner circumference wall surface 31 of the rotating drum 3, and the clothing 6 in the direction indicated by the arrow P. After performing the light irradiation for a predetermined time period, the control unit finishes the light irradiation at step S1002. As described above, the inner circumference wall surface of the water tub 2 and the outer circumference wall surface and inner circumference wall surface 31 of the rotating drum 3, to which the silver ions have been applied, are irradiated with the light after having finished the operation, thereby enabling the sterilization treatment for the inner circumference wall surface of the water tub 2 and the outer circumference wall surface and inner circumference wall surface 31 of the rotating drum 3.

Meanwhile, in the one embodiment of the fabric structure treatment apparatus according to the present invention, after having taken out the clothing 6 from the rotating drum 3, the light irradiating unit 20 may irradiate with the light the inner circumference wall surface of the water tub 2 and the outer circumference wall surface and inner circumference wall surface 31 of the rotating drum 3 in the above-described tub inside sterilization process; and before taking out the clothing 6 from the rotating drum 3, the light irradiating unit 20 may irradiate with the light the inner circumference wall surface of the water tub 2 and the outer circumference wall surface and inner circumference wall surface 31 of the rotating drum 3 in the above-described tub inside sterilization process.

In a case where the light irradiation is started in the light irradiation dewatering process at step S700 and thereafter, the laundering operation is finished, the light irradiation may be stopped after a predetermined time period has passed since the laundering operation was finished. In addition, in a case where the light irradiation is started in the light irradiation drying process at step S900 and thereafter, the drying operation is finished, the light irradiation may be stopped after a predetermined time period has passed since the drying operation was finished. This allows, when it is desired to sterilize the tub inside, the tub inside to be efficiently irradiated with the light for the predetermined time period after the laundering operation or the drying operation has been finished and the clothing 6 has been taken out from the rotating drum 3. Moreover, because the light irradiation is stopped after the predetermined time period has passed, it is not necessary for a user to stand by until the light irradiation is finished. At this time, for example, by rotating the rotating drum 3, not only the inner circumference wall surface of the water tub 2 and the outer circumference wall surface and inner circumference wall surface 31 of the rotating drum 3 but also a space inside of the tub may be irradiated with the light in an even manner.

Further, the door unit 7 is opened, the clothing 6 is taken out from the rotating drum 3, the door unit 7 is closed, and thereafter, the light irradiation may be started. This allows the light irradiation to be started in a closed space after the clothing 6 has been taken out.

Furthermore, not only after the laundering operation or the drying operation has been finished but also at least after the rinsing process in which the silver ions are applied to the rotating drum 3 or the like has been finished, the door unit 7 is opened in order to take out the clothing 6 from the rotating drum 3 and thereafter, the light irradiation may be started with the door unit 7 remaining open. This allows the light irradiation to be performed even when a user does not close the door unit 7 after having taken out the clothing 6. At this time, in order to avoid any problem arising when a user is exposed to the light, it is preferable that the emitted light is visible light.

Meanwhile, in a case where the washing and drying machine, the drying-dedicated machine, or the like as the one embodiment of the fabric structure treatment apparatus according to the present invention is used only for a purpose of drying, the silver ion water may be previously applied to the fabric structure such as clothing, a futon, a rug, and the like, and during the drying process, the light irradiation may be performed with the fabric structure in the container unit. In addition, in a case where the washing and drying machine, the drying-dedicated machine, or the like as the one embodiment of the fabric structure treatment apparatus according to the present invention is used only for a purpose of dewatering, the silver ion water may be previously applied to the fabric structure such as clothing, a futon, a rug, and the like which contain water, and during the dewatering process, light irradiation may be performed with the fabric structure in the container unit.

### EXAMPLES

In one example of a sterilization method in which a fabric structure treatment apparatus according to the present invention is used, silver ions were applied to polyester which was used as one example of a material of which a fabric structure is composed and thereafter, the polyester was irradiated with various kinds of light, whereby presence or absence of a sterilization effect was investigated.

Specifically, test samples having the silver ions applied thereto were irradiated with light and thereafter, an antibacterial effect was tested.

The test samples used in this test were obtained by applying silver ion water to the polyester and drying the polyester. The silver ion water was prepared by using tap water in the Yao city and eluting the silver ions from silver electrodes into the water through electrolysis.

The test was conducted under three light irradiation conditions: irradiation by a white fluorescent lamp; irradiation by a black light; and no light irradiation. Silver amounts applied to surfaces of the test samples were 0 (without a process of applying the silver ion water), 5, 10, and 20 ng/cm². An antibacterial test was conducted by employing a photoirradiation film contact method of "Society of Industrial Technology for Antimicrobial Articles". In this method, a bacterial suspension containing bacteria of approximately 1.0 × 10⁵ CFU are adhered to a test sample, and after 24 hours, as an irradiation time, has passed, the number of bacteria is measured. As a kind of bacteria, Staphylococcus aureus was used. A white fluorescent lamp of 20W was used, and the samples were placed at a position where a light level (illuminance) in the vicinity of the samples, which were irradiated by the white fluorescent lamp, was 5000 lux. Similarly, a black light of 20W was used, and also in the case of irradiation by the black light, the samples were placed at the same position where the light level (illuminance) in the vicinity of the samples, which were irradiated by the white fluorescent lamp, was 5000 lux.

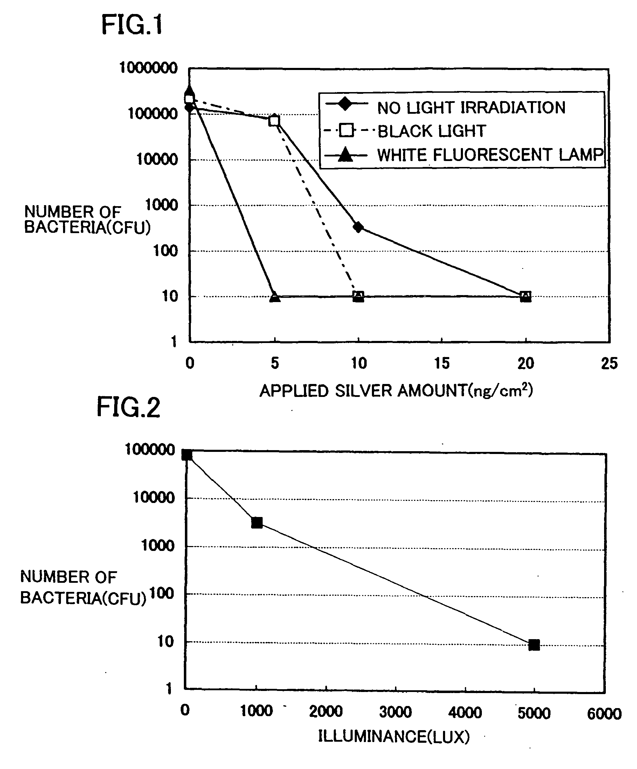

The result is shown in Table 1 and Fig. 1. Table 1 shows a relationship between the respective light irradiation conditions and the numbers of bacteria (unit: CFU) obtained after the test in the test samples having the respective silver amounts applied thereto. Fig. 1 is a graph showing a relationship between the respective silver amounts applied to the test samples and the numbers of bacteria existing in the test samples. The graph is plotted, assuming that an obtained value of the number of bacteria, which is less than or equal to a lower limit value (10 CFU), is 10.

**[Table 1]**

| | | Applied silver amount (ng/cm²) | | | |
|---|---|---|---|---|---|
| | | 0 | 5 | 10 | 20 |
| Light | No light irradiation | 1.4 × 10⁵ | 8.0×10⁴ | 3.4×10² | <10 |
| irradiation condition | Black light | 2.2 × 10⁵ | 7.1 × 10⁴ | <10 | <10 |
| | White fluorescent lamp | 3.4 × 10⁵ | < 10 | < 10 | < 10 |

As shown in Table 1 and Fig. 1, even under the condition of no light irradiation, when the applied silver amount was 20 ng/cm², sufficient bactericidal action was recognized. It is considered that evaporation residues of the silver ion water were attached to the surfaces of these test samples and the evaporation residues were dissolved again when the bacterial suspension was dripped down, whereby the bacterial action due to the silver ions was exhibited.

It can be seen that the bactericidal action is enhanced by the light irradiation. It was recognized that in the test samples having the silver amount of 10 ng/cm² applied thereto, the bactericidal action was enhanced by the irradiation of the black light and the white fluorescent lamp whereas under the condition that the small amount of silver, such as 5 ng/cm², was applied, the bactericidal action was further enhanced by the irradiation with the white fluorescent lamp mainly including visible light, rather than the irradiation of the black light mainly including near ultraviolet light.

In this test method, the evaluation wad made by adding the water in a form of the bacterial suspension to the above-mentioned dry samples. Therefore, it is considered that the evaporation residues of the silver ion water, adhering to the surfaces of the samples, were dissolved and turned into the silver ions, and the silver ions were irradiated with the light, whereby the bactericidal action was enhanced.

Basically, under a completely dry condition (for example, a condition under which a water activity is less than or equal to 0.5), bacteria die out. However, the environment in general is not dried to such an extent.

For example, even if laundry is sufficiently dried by a drying machine or the like, surfaces and crystals of molecules of the fabric contain moisture. Therefore, bacteria inhabit all over the general environment, even if it is not particularly humid. In particular, an amount of moisture contained in such a form in laundry dried at a low temperature is large.

Accordingly, as an evaluation method of bactericidal action in dry samples, addition of water is a general method, which is also performed in the JIS Z2801, JIS L1902, or the like, and is not an inappropriate method.

In addition, even if a dry material is targeted for sterilization in the present invention, moisture is present in the environment which allows bacteria to survive and therefore, the effect recognized in this test is exhibited. If the environment is completely dried to the extent that no bacteria can survive, sterilization is not needed and therefore, no problem arises.

In addition, in the above-mentioned test, the test samples having the silver amount of 5 ng/cm² applied thereto were irradiated with the light by using the white fluorescent lamp mainly including the visible light; an illuminance (lux) thereof was changed; and a change in the numbers of bacteria (unit: CFU) after the test was investigated. The result is shown in Fig. 2. Fig. 2 is a graph showing a relationship between illuminances of the white fluorescent lamp and the numbers of bacteria existing in the test samples.

As shown in Fig. 2, the number of bacteria obtained when no light irradiation was performed was 8.0 × 10⁴ CFU; the number of bacteria obtained when the illuminance was 1000 lux was 3.20 × 10³ CFU; and the number of bacteria obtained when the illuminance was 5000 lux was approximately less than or equal to 10 CFU. As mentioned above, a change in the bactericidal action attained by the present invention, which depended on the light intensity (illuminance), was recognized. It can be seen that the material having the silver ions applied thereto is irradiated with the light of at least greater than or equal to a predetermined illuminance, whereby the bactericidal action can be further enhanced.

In addition, when the graph was examined, a two-digit decrease in the number of bacteria, as compared with that obtained when no light irradiation was performed, resulted around 1900 lux. In the photoirradiation film contact method carried out this time, presence or absence of the antibacterial effect is determined based on whether or not a two-digit decrease in the number of bacteria is attained. This determination based on whether or not a two-digit decrease in the number of bacteria is attained is employed in the JIS Z2801, JIS L1902, or the like, and is used as a general criterion for determining the presence or absence of the antibacterial effect. Accordingly, from the viewpoint of enhancing the bactericidal action, it is desirable that irradiation of light of greater than or equal to a predetermined illuminance is performed and it is more desirable that an illuminance at a position targeted for the irradiation is greater than or equal to 1900 lux.

Further, in the above-mentioned test, test samples having a silver amount of 5 ng/cm² applied thereto were irradiated with light of an illuminance of 5000 lux by using the white fluorescent lamp mainly including the visible light; an irradiation time was changed (with silver and with light); and a change over time in the number of bacteria (unit: CFU), existing in the test samples, obtained after the test was investigated. In order to make a comparison, by using test samples, to each of which a silver amount of 5 ng/cm² was applied, which were not irradiated with the light, and which thereafter underwent a lapse of time (with silver and without light); by using test samples to which no silver ions were applied, which were irradiated with light of an illuminance of 5000 lux by using the white fluorescent lamp, and which thereafter underwent a lapse of time (without silver and with light); and by using test samples to which no silver ions were applied, which was not irradiated with light, and which thereafter underwent a lapse of time (without silver and without light), changes over time in the numbers of bacteria were investigated. The result was shown in Fig. 15. Fig. 15 is a graph showing a relationship between the lapses of time and the numbers of bacteria existing in the test samples.

As shown in Fig. 15, it can be seen that in a case of the test sample to each of which the silver amount of 5 ng/cm² was applied, which were irradiated with the light of the illuminance of 5000 lux by using the white fluorescent lamp mainly including the visible light; and for which the irradiation time was changed (with silver and with light), the number of bacteria was decreased for a very short time period of the irradiation time (one hour). Accordingly, the bactericidal action can be enhanced by performing the light irradiation during the operation or the like of the washing machine or the washing and drying machine. For example, it is preferable that the light irradiation is performed during the drying process in the washing and drying machine.

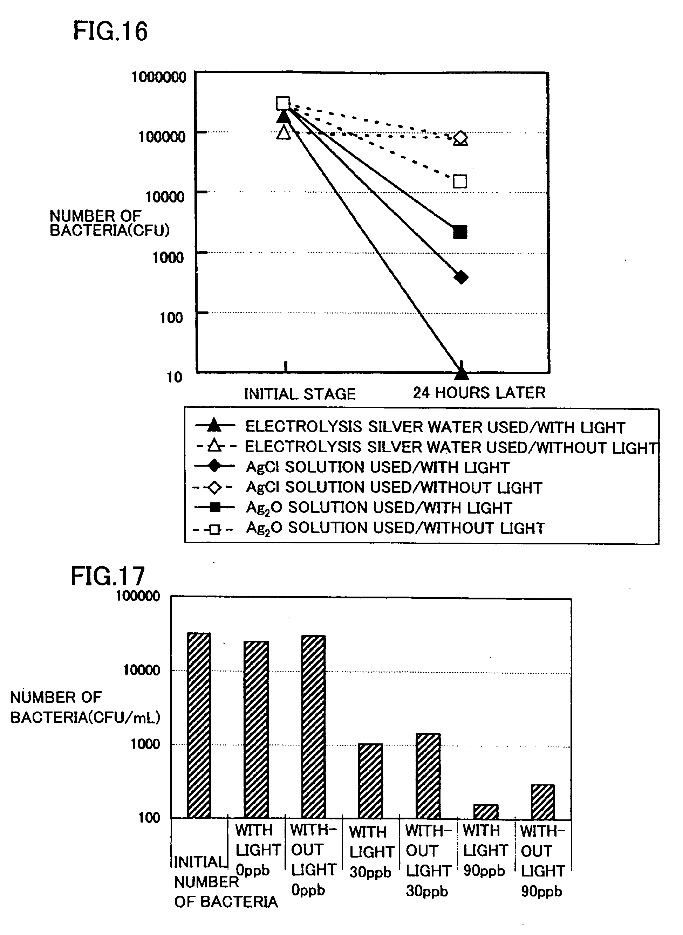

Furthermore, in the above-mentioned test, in a case where a method of generating the silver ions applied to test samples, which is different from the above-mentioned method, is employed, the numbers of bacteria (unit: CFU) obtained after the test were investigated. A test sample, which as similarly to in the method of generating the silver ions in the above-mentioned test, was prepared by using silver ion water (electrolysis silver ion water) obtained by eluting the silver ions into water through electrolysis (electrolyzation) from the silver electrodes immersed in the tap water, was used; a test sample, which was prepared by using silver ion water (AgCl solution) obtained by dissolving silver chloride (AgCl) as a reagent in water, was used; and a test sample, which was prepared by using silver ion water (Ag₂O solution) obtained by dissolving silver oxide (Ag₂O) as a reagent, was used. Each of the test samples was irradiated with the light of an illuminance of 5000 lux by using the white fluorescent lamp mainly including the visible light; each of the test samples underwent the irradiation for 24 hours; and thereafter, the number of bacteria was investigated. In order to make a comparison, each of the test samples was irradiated with no light; each of the test samples underwent a lapse of 24 hours; and thereafter, the number of bacteria was investigated. The result is shown in Fig. 16. Fig. 16 is a graph showing a relationship between a kind of each of the methods of generating the silver ions to be applied and the number of bacteria existing in each of the test samples.

As shown in Fig. 16, it can be seen that in the test sample to which the silver ions were applied by using the electrolysis silver ion water, an extent to which the number of bacteria was decreased after the light irradiation is large and the number of bacteria was decreased to the largest extent after the light irradiation, as compared with the test samples to which the silver ion water was applied by using the AgCl solution and the Ag₂O solution.

Conducted next was a test in which silver ion water having a bacterial suspension added therein was irradiated with light. As a light source for the light irradiation, the white fluorescent lamp was used and placed such that an illuminance at a position of the sample was 10,000 lux. The light irradiation was performed for 10 minutes and Pseudomonas aeruginosa was used as a kind of bacteria. The silver ion water containing a predetermined number of the bacteria was put into a container through which the visible light can pass and the light irradiation was performed for 10 minutes. In order to conduct at the same time a test in which no light irradiation is performed, the same silver ion water containing the same bacteria was put into the same container, the container was covered with aluminum foil so as to completely block out the light, and under the same conditions other than the above-mentioned conditions, the test was conducted. A temperature around the sample during the test was 26°C. In addition, in this test, tap water which was subjected to autoclave sterilization was used and silver ion water obtained by eluting the silver ions into water through electrolysis (electrolyzation) from silver electrodes immersed in the tap water was used. Silver ion concentrations were 0, 30, and 90 ppb.

The result obtained from this test is shown in Fig. 17 and Table 2. Fig. 17 is a graph showing a relationship between each of the silver ion concentrations with and without the light irradiation and the number of bacteria. In Table 2, in the relationship between each of the silver ion concentrations with and without the light irradiation and the number of bacteria, the numbers of bacteria obtained when the light irradiation was performed are shown with % in a case where the number of bacteria obtained when no light irradiation was performed is supposed to be 100%.

**[Table 2]**

| | Silver ion concentration (ppb) | | |
|---|---|---|---|
| | 0 | 30 | 90 |
| With light irradiation | 83% | 73% | 53% |
| Without light irradiation | 100% | 100% | 100% |

It can be seen from the result shown in Fig. 17 and Table 2 that whereas when the silver ion concentration was 0 ppb, the number of bacteria remained almost unchanged from the initial number of bacteria regardless of presence or absence of the light irradiation, the sterilization effect due to the silver as well as the sterilization effect due to the light irradiation increased in accordance with an increase in the silver ion concentration.

Note that in this test, an ultraviolet intensity included in the light emitted from the light source was 2 µW/cm² and irradiation with ultraviolet light alone attained no sterilization effect.

As described above, the effect of sterilizing the water can be enhanced by irradiating the silver ion water with the light. Accordingly, in the washing machine as the fabric structure treatment apparatus according to the present invention, the silver ions are caused to be contained in water used for washing and rinsing and the water is irradiated with the light by the light irradiating unit, whereby the washing water and rinsing water can be effectively sterilized. Such an effect is particularly effective when water, such as bathwater, containing bacteria, is used for laundering.

In addition, it is considered that even if the test samples which are not dried and have the silver ion water kept therein are irradiated with the light, similar enhancement of the bactericidal action can be attained because the silver ions are contained in the silver ion water as similarly to the conditions under which this test was conducted. Accordingly, even when a fabric structure such as a cloth immersed in the silver ion water is irradiated with the light, the bactericidal action can be enhanced. For example, it is considered that even when laundry, to which the silver ion water has been applied during the rinsing process, and components, such as the washing tub and the water tub inside of the washing machine, to which the silver ion water has adhered are irradiated with the light, the bactericidal action can be enhanced.

The described embodiments and examples are to be considered in all respects only as illustrative and not restrictive. It is intended that the scope of the invention is, therefore, indicated by the appended claims rather than the foregoing description of the embodiments and examples and that all modifications and variations coming within the meaning and equivalency range of the appended claims are embraced within their scope.

### INDUSTRIAL APPLICABILITY

A fabric structure treatment apparatus according to the present invention is applicable to, for example, a washing machine, a washing and drying machine, a drying machine, or the like as a treatment apparatus used for a fabric structure such as clothing, a futon, a rug, etc.

## Claims

1. A fabric structure treatment apparatus (100) comprising:
a container unit (2, 3) for containing, inside thereof, a fabric structure (6) to which silver ions have been applied; and
a light irradiating unit (20) for irradiating with light the fabric structure (6) to which the silver ions have been applied.

2. The fabric structure treatment apparatus (100) according to claim 1, wherein the silver ions are silver ions generated through electrolysis.

3. A fabric structure treatment apparatus (100) comprising:
a container unit (2, 3) for containing, inside thereof, a fabric structure (6);
a silver ion applying unit (40) for applying silver ions to the container unit (2, 3) and/or the fabric structure (6) contained in the container unit (2, 3); and
a light irradiating unit (20) for irradiating with light the container unit (2, 3) and/or the fabric structure (6), to which the silver ions have been applied by the silver ion applying unit (40).

4. The fabric structure treatment apparatus (100) according to claim 3, wherein the light irradiating unit (20) emits light including visible light.

5. The fabric structure treatment apparatus (100) according to claim 3, wherein a washing process (S200) of washing the fabric structure (6) is performed and after the washing process (S200), the light irradiating unit (20) irradiates with the light the container unit (2, 3) and/or the fabric structure (6), to which the silver ions have been applied by the silver ion applying unit (40).

6. The fabric structure treatment apparatus (100) according to claim 3, wherein a rinsing process (S300, S400) of rinsing the fabric structure (6) is performed; during the rinsing process (S400), the silver ion applying unit (40) applies the silver ions to the container unit (2, 3) and/or the fabric structure (6); and after the rinsing process (S300), the light irradiating unit (20) irradiates with the light the container unit (2, 3) and/or the fabric structure (6), to which the silver ions have been applied by the silver ion applying unit (40).

7. The fabric structure treatment apparatus (100) according to claim 3, wherein a dewatering process (S600, S700) of removing water from the fabric structure (6) is performed; and during the dewatering process (S700) and/or after the dewatering process (S600), the light irradiating unit (20) irradiates with the light the container unit (2, 3) and/or the fabric structure (6), to which the silver ions have been applied by the silver ion applying unit (40).

8. The fabric structure treatment apparatus (100) according to claim 3, wherein a drying process (S800, S900) of drying the fabric structure (6) is performed; and during the drying process (S900) and/or after the drying process (S800), the light irradiating unit (20) irradiates with the light the container unit (2, 3) and/or the fabric structure (6), to which the silver ions have been applied by the silver ion applying unit (40).

9. The fabric structure treatment apparatus (100) according to claim 8, wherein the drying process (S800, S900) is performed at a low temperature.

10. The fabric structure treatment apparatus (100) according to claim 9, wherein the drying process (S800, S900) is performed by using a heat pump unit.

11. The fabric structure treatment apparatus (100) according to claim 3, wherein after the fabric structure (6) has been taken out from the container unit (2, 3), the light irradiating unit (20) irradiates with the light the container unit (2, 3).

12. The fabric structure treatment apparatus (100) according to claim 3, wherein the silver ions are silver ions generated through electrolysis.

13. A fabric structure treatment apparatus (100) which uses water containing silver ions and comprises a light irradiating unit (20) for irradiating the water with light.

14. The fabric structure treatment apparatus according to claim 13, wherein the silver ions are silver ions generated through electrolysis.
